(19) Europäisches Patentamt
European Patent Office
Office européen des brevets

(11) Publication number: 0 061 763
B1

(12) **EUROPEAN PATENT SPECIFICATION**

(45) Date of publication of patent specification: **18.06.86**

(21) Application number: **82102644.0**

(22) Date of filing: **29.03.82**

(51) Int. Cl.$^4$: **C 07 D 205/08,**
C 07 D 403/12,
C 07 D 407/12,
C 07 D 407/14,
C 07 D 409/12,
C 07 D 409/14,
C 07 D 417/12,
C 07 D 417/14, A 61 K 31/395

# (54) 4-Substituted mercapto-2-oxo-1-azetidinesulfonic acid salts and process for their preparation.

(30) Priority: **30.03.81 US 249258**
**16.07.81 US 283751**

(43) Date of publication of application:
**06.10.82 Bulletin 82/40**

(45) Publication of the grant of the patent:
**18.06.86 Bulletin 86/25**

(84) Designated Contracting States:
**AT BE CH DE FR GB IT LI LU NL SE**

(56) References cited:
**EP-A-0 021 678**
**EP-A-0 053 815**

(73) Proprietor: **E.R. Squibb & Sons, Inc.**
**World Headquarters P. O. Box 4000**
**Princeton New Jersey 08540 (US)**

(72) Inventor: **Treuner, Uwe D.**
**Müllerstrasse 5**
**D-8400 Regensburg (DE)**
Inventor: **Denzel, Theodor**
**Lessingstrasse 12**
**D-8400 Regensburg (DE)**
Inventor: **Breuer, Hermann**
**Sauerzapfstrasse 5**
**D-8411 Schönhofen (DE)**
Inventor: **Cimarusti, Christopher M.**
**278 Wargo Road**
**Pennington New Jersey (US)**

(74) Representative: **Vossius Vossius Tauchner**
**Heunemann Rauh**
**Siebertstrasse 4 P.O. Box 86 07 67**
**D-8000 München 86 (DE)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

In EP—A—53815 and EP—A—21678 there are described 1-sulfo-2-oxeoazetidinederivatives having an antibacterial effect.

Antibacterial activity is exhibited by β-lactams having the formula I

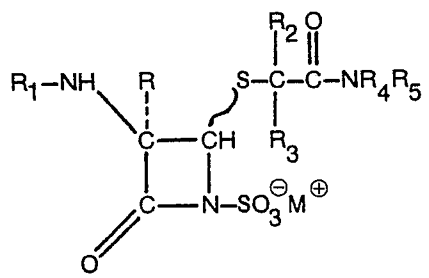

(I)

wherein R is hydrogen or methoxy;

$R_1$ is acyl, derived from a carboxylic acid, or hydrogen

$R_2$ and $R_3$ are each independently hydrogen or $C_1$—$C_{10}$ alkyl, or $R_2$ and $R_3$ when taken together with the carbon atom to which they are attached form a $C_3$—$C_7$ cycloalkyl group;

$R_4$ and $R_5$ are the same or different and each is hydrogen, $C_1$—$C_{10}$-alkyl or phenyl or phenyl-$C_1$—$C_{10}$-alkyl wherein the phenyl group may be substituted with 1, 2 or 3 amino, halogen, hydroxyl, trifluormethyl, $C_1$—$C_4$ alkyl or $C_1$—$C_4$ alkoxy groups;

$M^{\oplus}$ is hydrogen or a cation.

The terms "alkyl" and "alkoxy" refer to both straight and branched chain groups.

The term "$C_3$—$C_7$-"cycloalkyl" refers to groups having 3,4,5,6 or 7 carbon atoms.

The term "halogen" refers to fluorine, chlorine, bromine and iodine.

The term "acyl derived from a carboxylic acid" defines radicals derived from a carboxylic acid by removal of the hydroxyl group. Certain acyl groups are, of course, preferred but this preference should not be viewed as a limitation of the scope of this invention. Exemplary acyl groups are those acyl groups which have been used in the past to acylate β-lactam antibiotics including 6-aminopenicillanic acid and derivatives and 7-aminocephalosporanic acid and derivatives; see, for example, *Cephalosporins and Penicillins,* edited by Flynn, Academic Press (1972), DE-A-2,716,677, published October 10 1978, BE—A—867,994, published December 11, 1978, US—A—4,152,432, issued May 1, 1979, US—A—3,971,778, issued July 27, 1976, US—A—4,172,199, issued October 23, 1979, and GB—A—1,348,894, published March 27, 1974. The following list of acyl groups is presented to further exemplify the term "acyl". Exemplary acyl groups are:

(a) Aliphatic groups having the formula

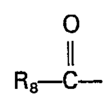

wherein $R_8$ is alkyl; cycloalkyl; alkoxy; alkenyl; cycloalkenyl; cyclohexadienyl; or alkyl or alkenyl substituted with one or more halogen, cyano, nitro, amino, mercapto, alkylthio, or cyanomethylthio groups.

The terms "alkyl, alkoxy, alkenyl" refer to both straight and branched chain groups; those having up to 10 C-atoms are preferred. "Cycloalkenyl" refers to groups having 3—7 C-atoms. These definitions apply throughout the specification unless they are not otherwise limited.

(b) Carbocyclic aromatic groups having the formula

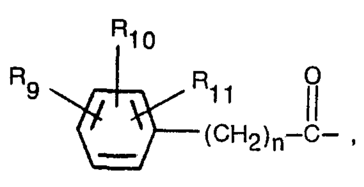

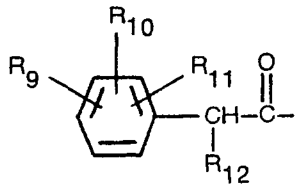

,

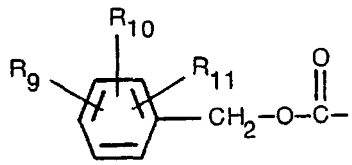

,

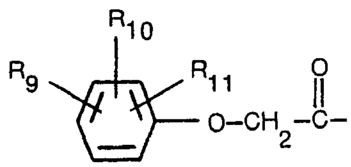

,

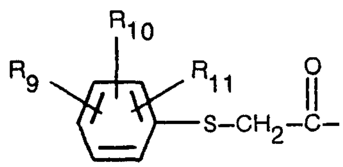

or

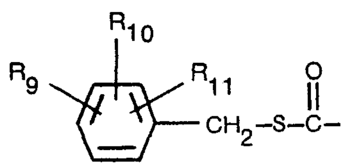

wherein n is 0, 1, 2 or 3; $R_9$, $R_{10}$, and $R_{11}$ each is independently hydrogen, halogen, hydroxyl, nitro, amino, cyano, trifluoromethyl, alkyl of 1 to 4 carbon atoms, alkoxy of 1 to 4 carbon atoms or aminomethyl; and $R_{12}$ is amino, hydroxyl, a carboxyl salt, protected carboxyl, formyloxy, a sulfo salt, a sulfoamino salt, azido, halogen, hydrazino, alkylhydrazino, phenylhydrazino, or [(alkylthio)thioxomethyl]thio.

Preferred carbocyclic aromatic acyl groups include those having the formula

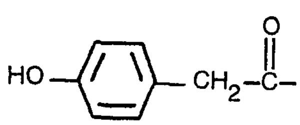

,

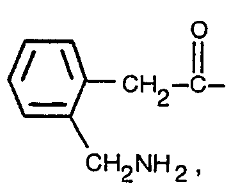

,

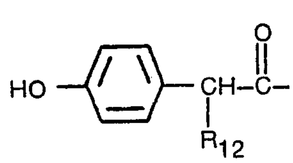

($R_{12}$ is preferably a carboxyl salt or sulfo salt) and

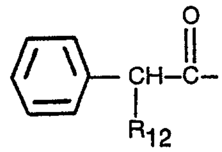

($R_{12}$ is preferably a carboxyl salt or sulfo salt).

(c) Heteroaromatic groups having the formula

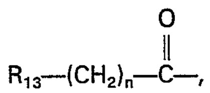

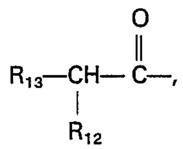

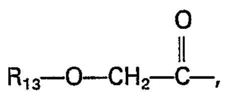

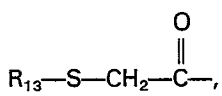 or

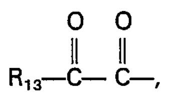

wherein n is 0, 1, 2 or 3; $R_{12}$ is as defined above; and $R_{13}$ is a substituted or unsubstituted 5-, 6- or 7-membered heterocyclic ring containing 1, 2, 3 or 4 (preferably 1 or 2) nitrogen, oxygen and sulfur atoms. Exemplary heterocyclic rings are thienyl, furyl, pyrrolyl, pyridinyl, pyrazolyl, pyrazinyl, thiazolyl, morpholinyl, pyrimidinyl and tetrazolyl. Exemplary substituents are halogen, hydroxyl, nitro, amino, cyano, trifluoromethyl, alkyl of 1 to 4 carbon atoms, alkoxy of 1 to 4 carbon atoms or

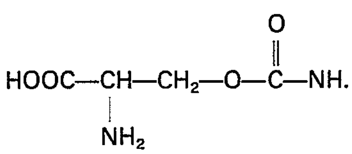

Preferred heteroaromatic acyl groups includes those groups of the above formulas wherein $R_{13}$ is 2-amino-4-thiazolyl, 2-amino-5-halo-4-thiazolyl, 4-aminopyrimidin-2-yl, 5-amino-1,2,4-thiadiazol-3-yl, 2-thienyl, 2-furanyl, or 6-aminopyridin-2-yl.

(d) [[(4-Substituted-2,3-dioxo-1-piperazinyl)carbonyl]amino]arylacetyl groups having the formula

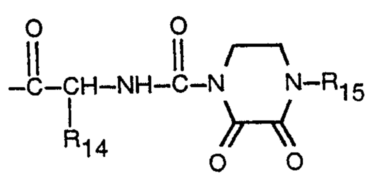

wherein $R_{14}$ is an aromatic group (including carbocyclic aromatics such as those of the formula

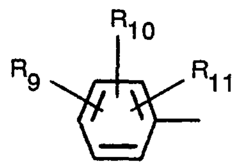

and heteroaromatics as included within the definition of $R_{13}$); and $R_{15}$ is alkyl, substituted alkyl (wherein the alkyl group is substituted with one or more halogen, cyano, nitro, amino or mercapto groups), arylmethyleneamino (*i.e.,* $—N{=}CH—R_{14}$ wherein $R_{14}$ is as defined above), arylcarbonylamino (*i.e.,*

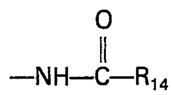

wherein $R_{14}$ is as defined above) or alkylcarbonylamino.

Preferred [[(4-substituted-2,3-dioxo-1-piperazinyl)carbonyl]amino]arylacetyl groups include those wherein $R_{15}$ is ethyl, phenylmethyleneamino or 2-furylmethyleneamino.

(e) (Substituted oxyimino)arylacetyl groups having the formula

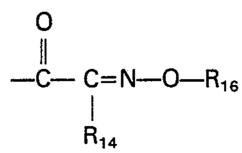

wherein $R_{14}$ is as defined above and $R_{16}$ is hydrogen, alkyl, cycloalkyl, alkylaminocarbonyl, arylamino-carbonyl (*i.e.,*

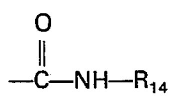

wherein $R_{14}$ is as defined above) or substituted alkyl (wherein the alkyl group is substituted with 1 or more halogen, cyano, nitro, amino, mercapto, alkylthio, aromatic group (as defined by $R_{14}$), carboxyl (including salts thereof), amido, alkoxycarbonyl, phenylmethoxycarbonyl, diphenylmethoxycarbonyl, hydroxyalkoxy-phosphinyl, dihydroxyphosphinyl, hydroxy (phenylmethoxy)phosphinyl, or dialkoxyphosphinyl substituents).

Preferred (substituted oxyimino)arylacetyl groups include those wherein $R_{14}$ is 2-amino-4-thiazolyl. Also preferred are those groups wherein $R_{16}$ is methyl, ethyl, carboxymethyl, 1-carboxy-1-methylethyl or 2,2,2-trifluoroethyl

(f) (Acylamino)arylacetyl groups having the formula

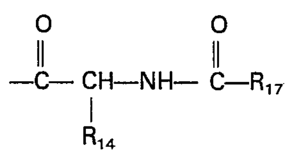

wherein $R_{14}$ is as defined above and $R_{17}$ is

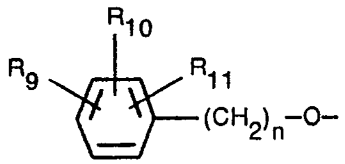 , amino, alkylamino, (cyanoalkyl)amino,

amido, alkylamido, (cyanoalkyl)amido,

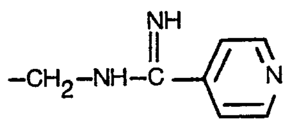 , 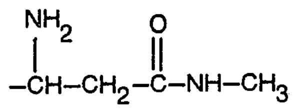 ,

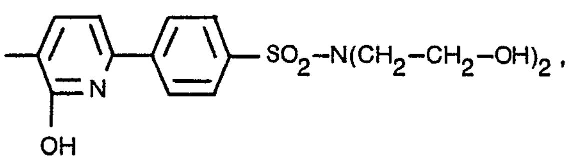 , 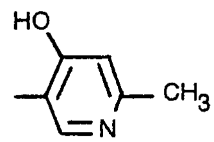 ,

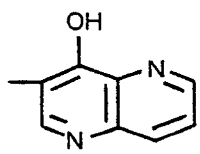 , or 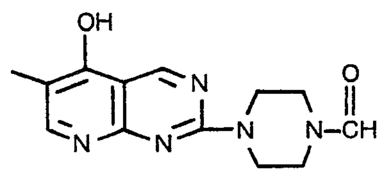 .

Preferred (acylamino)arylacetyl groups of the above formula include those groups wherein $R_{17}$ is amino, or amido. Also preferred are those groups wherein $R_{14}$ is phenyl or 2-thienyl.

(g) [[[3-Substituted-2-oxo-1-imidazolidinyl]carbonyl]amino]arylacetyl groups having the formula

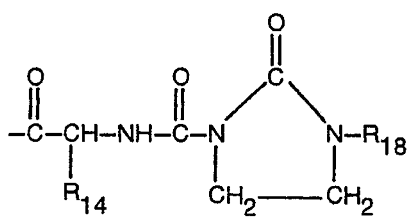

wherein $R_{14}$ is as defined above and $R_{18}$ is hydrogen, alkylsulfonyl, arylmethyleneamino (*i.e.,* —N=CH—$R_{14}$ wherein $R_{14}$ is as defined above),

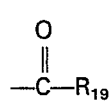

(wherein $R_{19}$ is hydrogen, alkyl or halogen substituted alkyl), aromatic group (as defined by $R_{14}$ above), alkyl or substituted alkyl (wherein the alkyl group is substituted with one or more halogen, cyano, nitro, amino or mercapto groups).

Preferred [[3-substituted-2-oxo-1-imidazolidinyl]carbonyl]amino]arylacetyl groups of the above formula include those wherein $R_{14}$ is phenyl or 2-thienyl. Also preferred are those groups wherein $R_{18}$ is hydrogen, methylsulfonyl, phenylmethyleneamino or 2-furylmethyleneamino.

The term "cation", as used throughout the specification, refers to any positively charged atom or group of atoms. The "—$SO_3^{\ominus}M^{\oplus}$" substituent on the nitrogen atom of the β-lactams of this invention encompasses all sulfonic acid salts. Pharmaceutically acceptable salts are, of course, preferred, although

other salts are also useful in purifying the products of this invention or as intermediates for the preparation of pharmaceutically acceptable salts. The cationic portion of the sulfonic acid salts of this invention can be obtained from either organic or inorganic bases. Such cationic portion includes, e.g. the following ions: ammonium; substituted ammonium, such as alkylammonium (*e.g.,* tetra-n-butylammonium, referred to hereinafter as tetrabutylammonium); alkali metal, such as lithium, sodium and potassium; alkaline earth metal, such as calcium and magnesium; pyridinium; dicyclohexylammonium; hydrabaminium; benzathinium; N-methyl-D-glucaminium.

As set forth in formula I, and in the definitions following formula I, $M^{\oplus}$ can be hydrogen. Such compounds are often referred to in the art as "inner salts" by virtue of a positive and negative charge in the molecule.

This invention is directed to those β-lactams which have been described above, wherein the stereochemistry at the chiral center in the 3-position of the β-lactam nucleus is the same as the configuration at the carbon atom in the 6-position of naturally occurring penicillins (*e.g.,* penicillin G) and as the configuration at the carbon atom in the 7-position of naturally occurring cephamycins (*e.g.,* cephamycin C).

The β-lactams of formula I have activity against a range of gram-negative and gram-positive organisms. The compounds of this invention can be used as agents to combat bacterial infections (including urinary tract infections and respiratory infections) in mammalian species, such as domesticated animals (*e.g.,* dogs, cats, cows, horses, and the like) and humans.

For combating bacterial infections in mammals a compound of this invention can be administered to a mammal in need thereof in an amount of 1.4 mg/kg/day to 350 mg/kg/day, preferably 14 mg/kg/day to 100 mg/kg/day. All modes of administration which have been used in the past to deliver penicillins and cephalosporins to the site of the infection are also contemplated for use with the novel β-lactams of this invention. Such methods of administration include oral, intravenous, intramusuclar, and as a suppository.

The compound of formula I can be prepared from an azetidine having the formula

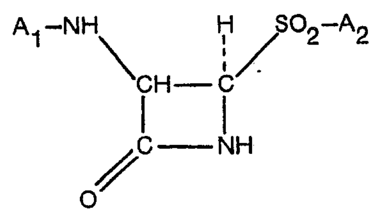
,
II

wherein $A_1$ is a nitrogen protecting group, *e.g.,* triphenylmethyl, and $A_2$ is an alkyl or aryl group. Reaction of an azetidine of formula II with a thiolate having the formula III

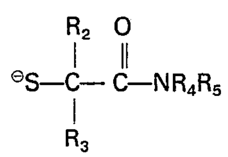
(III)

yields a mixture of diastereomers of the formulas IVa and IVb

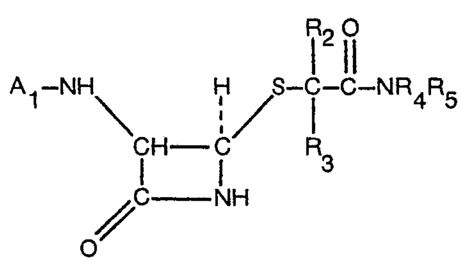
(IVa)

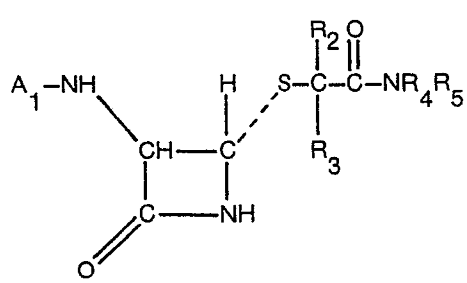
(IVb)

Separation of the above diastereomers can be accomplished using conventional chromatographic or fractional crystallization techniques.

The addition of a sulfo ($SO_3^{\ominus}$) group to the 1-position of a compound of formula IVa or IVb or the R = methoxy counterpart yields the corresponding compound having the formula V

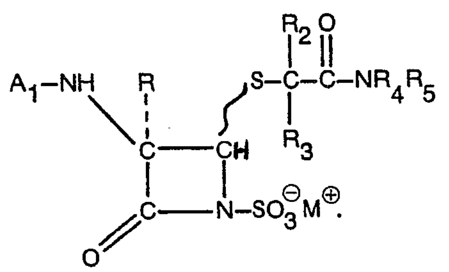

(V)

Introduction of the sulfo substituent can be accomplished by sulfonation as for example by reacting a compound of formula IVa or IVb or the R = methoxy counterpart with a complex of pyridine and sulfur trioxide. The reaction can be run in an organic solvent or in a mixture of organic solvents, preferably a mixture of a polar sovlent such as dimethylformamide and a halogenated hydrocarbon such as dichloromethane. This reaction yields a compound of formula V wherein $M^{\oplus}$ is pyridinium ion. Instead of using a pre-formed complex of pyridine and sulfur trioxide, the complex can be formed *in situ, e.g.,* using chlorosulfonyltrimethylsilyl ester and pyridine as reagents. Alternatively, a complex of dimethylformamide-sulfur trioxide, 2-picoline-sulfur trioxide or 2,6-lutidine sulfur trioxide can be used.

Using conventional techniques (*e.g.,* ion-exchange resins, crystallization, or ion-pair extraction) the pyridinium salt formed by the above procedure can be converted to other salts. These techniques can also be used to convert the products of formula I, or any of the intermediates described herein, to other salts.

A second method for introducing the sulfo group to the 1-position of an azetidine of formula IVa or IVb comprises first silylating the compound and then subjecting the silated compound to a silyl interchange reaction. Exemplary silylating agents are monosilyltrifluoroacetamide, trimethylsilylchloride/triethylamine, and bis-trimethylsilyltrifluoroacetamide, and an exemplary reagent useful for the silyl interchange reaction is trimethylsilyl chlorosulfonate.

Deprotection of an azetidine of formula V yields a zwitterion having the formula VI

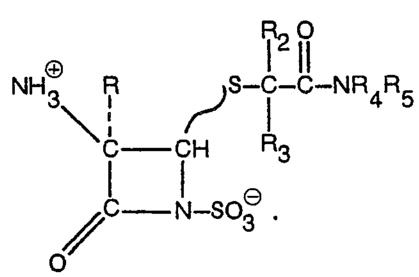

VI

The deprotection techniques used are conventional, and will depend on the particular protecting group ($A_1$) present. Treatment with acid (*e.g.* formic acid or trifluoroacetic acid) cleaves a triphenylmethyl or a *t*-butoxycarbonyl protecting group. Treatment with hydrogen (using a catalyst such as palladium) cleaves a benzyloxycarbonyl protecting group. Treatment with phosgene or phosphorous pentachloride cleaves an amide protecting group. The zwitterions of formula VI are novel intermediates, and as such, constitute an integral part of this invention.

Conventional acylation techniques can be used to prepare the products of formula I (wherein R is hydrogen) from a zwitterion of formula VI. Exemplary acylation techniques include reaction with a carboxylic acid ($R_1$—OH), or corresponding carboxylic acid halide or carboxylic acid anhydride. The reaction with a carboxylic acid proceeds most readily in the presence of a carbodiimide and a substance capable of forming an active ester *in situ* such as N-hydroxybenzotriazole. In those instances when the acyl group ($R_1$) contains reactive functionality (such as amino or carboxyl groups) it may be necessary to first protect those functional groups, then carry out the acylation reaction, and finally deprotect the resulting product.

Variations of the above described synthetic route (some of which are described in the examples presented below) will be apparent to the practitioner of this invention. For example, a compound of formula IVa or IVb can be deprotected and then protected with a different amino protecting group before proceeding with the remaining synthetic procedure.

The β-lactams of formula I wherein R is methoxy can be prepared from the corresponding compound of formula I wherein R is hydrogen. Halogenation of the amide nitrogen of a non-methoxylated compound of formula I yields, in situ, an intermediate having the formula VII

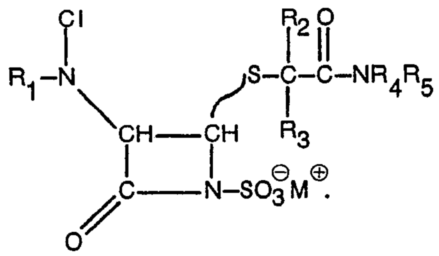

(VII)

Reaction of an intermediate of formula VII with a methoxylating agent, *e.g.,* an alkali metal methoxide yields a product of formula I wherein R is methoxy. The reaction can be run in an organic solvent, *e.g.,* a polar organic solvent such as dimethylformamide, at a reduced temperature.

An alternative synthesis for preparing the compounds of formula I wherein R is methoxy comprises first alkoxylating a compound of formula IVa or IVb wherein $A_1NH$ is a carbamate (*e.g.,* $A_1$ is benzyloxycarbonyl) and then introducing a sulfo group in the 1-position of the resulting compound. Deprotection and acylation, using the procedures described above, yields the products of formula I wherein R is alkoxy.

Alternative syntheses for the preparation of the products of this invention are available. For example, compounds of formula I wherein the group

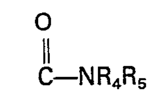 is 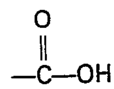

can be used as intermediates to obtain corresponding products of formula I. Standard coupling reactions are employed.

The following examples are specific embodiments of this invention.

The compounds of the preparative examples I, II and III are not claimed; the process for their preparation only serves to illustrate the preparation of the following examples.

Preparative Example I

(3R-*cis*)-[(3-Amino-2-oxo-1-sulfo-4-azetidinyl)thio]acetic acid, methyl ester

*A)* (3R-*cis*)-[[2-Oxo-3-[(triphenylmethyl)amino]-4-azetidinyl]thio]acetic acid, methyl ester

(3R-*cis*)-[2-Oxo-3-[(triphenylmethyl)amino]-4-azetidinyl]methyl sulfone (20.35 g) is dissolved in 100 ml of acetone and 6.4 g of the sodium salt of mercaptoacetic acid, methyl ester, is added in small portions. After the addition is complete, the solution is stirred for 3 hours at 40°C. The acetone is distilled off and the residue is dissolved in ethyl acetate and extracted three times with brine. The organic layer is dried over $Na_2SO_4$ and stripped off. The remaining oil is chromatographed on 400 g of silica, eluting with 9:1 chloroform/ethyl acetate, and yielding 7.3 g of the *cis* product, 6.4 g of the corresponding *trans* product and 2.6 g of a mixture of the *cis* and *trans* products.

*B)* (3R-*cis*)-[[2-Oxo-1-sulfo-3-[(triphenylmethyl)amino]-4-azetidinyl]thio]acetic acid, methyl ester, tetrabutylammonium salt

(3R-*cis*)-[[2-Oxo-3-[(triphenylmethyl)amino]-4-azetidinyl]thio]acetic acid, methyl ester (0.43 g) and 3.6 mmole of a complex of pyridine-sulfur trioxide are dissolved in 10 ml of dimethylformamide (dried over molecular sieves) and stirred for 3 hours at 60°C and 8 hours at room temperature. Dimethylformamide is stripped off *in vacuo* and the oily residue is dissolved in a mixture of 60 ml of 0.5 M potassium phosphate buffer pH 5.5 and 30 ml of dichloromethane. Tetrabutylammonium hydrogen sulfate (1 mmole) is added and the organic layer is separated. The aqueous phase is again extracted with 50 ml of dichloromethane. The combined organic layers are dried and dichloromethane is distilled off, yielding 0.6 g of the title compound.

*C)* (3R-*cis*)-[(3-Amino-2-oxo-1-sulfo-2-azetidinyl)thio]acetic acid, methyl ester

(3R-*cis*)-[[2-Oxo-1-sulfo-3-[(triphenylmethyl)amino]-4-azetidinyl]thio]acetic acid, methyl ester, tetrabutylammonium salt (0.3 g) is dissolved in 1 ml of dichloromethane, 8 ml of 98% formic acid is added, and the mixture is stirred for 2 hours. The solution is poured into 30 ml of ether, yielding 0.06 g of the title compound, melting point 103—105°C, *dec.*

Preparative Example II

[(3R-*cis*)(R*)]-[[3-[[[[(4-Ethyl-2,3-dioxo-1-piperazinyl)carbonyl]amido]phenylacetyl]amino]-2-oxo-1-sulfo-4-azetidinyl]thio]acetic acid; methyl ester, potassium salt

(3R-*cis*)-[(3-Amino-2-oxo-1-sulfo-4-azetidinyl)thio]acetic acid, methyl ester (542 mg; see example 1), 444 mg of (R)-α-[[(4-ethyl-2,3-dioxo-1-piperazinyl)carbonyl]amino]benzeneacetic acid, 202 mg of triethylamine and 350 mg of N-hydroxybenzotriazole are dissolved in 10 ml of dimethylformamide. After cooling in an ice-bath, 412 mg of dicyclohexylcarbodiimide dissolved in 3 ml of tetrahydrofuran is added dropwise while stirring. After 90 minutes the dicyclohexylurea which forms is filtered off and dimethylformamide is distilled off *in vacuo* (bath temperature 50°C). The remaining oil is dissolved in 30 ml of acetone and 0.7 g of potassium perfluorobutane sulfonate is added, causing 0.7 g of the title compound to precipitate. Purification is performed by column chromatography on HP-20 resin using water and 9:1 water/acetone as eluents. The yield of title compound, melting point 167°C, *dec.*, is 0.27 g after lyophilization.

Preparative Example III

[3R-[3α,4α(Z)]]-3-[[(2-Amino-4-thiazolyl)(methoxyimino)acetyl]amino]-4-(methylthio)-2-oxo-1-azetidinesulfonic acid, potassium salt

*A)* (3R-*cis*)-4-(Methylthio)-3-[(triphenylmethyl)amino]-2-oxo-1-azetidinesulfonic acid, tetrabutylammonium salt

(3R-*cis*)-4-(Methylthio)-3-[(triphenylmethyl)amino]-2-azetidinone (1.87 g and 1.2 g of a complex of pyridine and sulfur trioxide are dissolved in 15 ml of dimethylformamide and heated for 10 minutes to 70°C. The clear solution is then cooled to 5°C, stirred for 4 hours and poured into 100 ml of ice water. Tetrabutylammonium hydrogen sulfate is added and the pH is adjusted to 5.5. Extraction with dichloromethane yields 2.6 g of the title compound as an oil.

*B)* (3R-*cis*)-3-Amino-4-(methylthio)-2-oxo-1-azetidinesulfonic acid, inner salt

(3R-*cis*)-4-(Methylthio)-3-[(triphenylmethyl)amino]-2-oxo-1-azetidinesulfonic acid, tetrabutylammonium salt (2.4 g) is dissolved in 10 ml of dichloromethane and 2.5 ml of formic acid is added. The solution is stirred at −10°C for 30 minutes and poured into 100 ml of ether. Crude product (0.7 g) precipitates and is immediately used in part C.

*C)* [3R-[3α,4α(Z)]]-3-[[(2-Amino-4-thiazolyl)(methoxyimino)acetyl]amido]-4-[[(chlorophenyl)methyl]thio]-2-oxo-1-azetidinesulfonic acid, potassium salt

(3R-*cis*)-3-Amino-4-(methylthio)-2-oxo-1-azetidinesulfonic acid, inner salt (420 mg), 404 mg of (Z)-2-amino-α-(methoxyimino)-4-thiazoleacetic acid, 202 mg of triethylamine and 300 mg of N-hydroxybenzotriazole are dissolved in 10 ml of dimethylformamide and stirred at −5°C. A solution of 412 mg of dicyclohexylcarbodiimide in 5 ml of tetrahydrofuran is added dropwise. After stirring for 1 hour dicyclohexylurea is filtered off and the solvent is distilled off *in vacuo*. The oily residue is dissolved in acetone, 680 mg of potassium perfluorobutanesulfonate is added and 530 mg of crude product precipitates. The crude product is chromatographed on HP20, using water as eluent, and yielding 130 mg of the title compound, melting point 193°C, *dec.*

Examples 1—5

Following the procedure of Example I, but substituting the compound listed in column I for the sodium salt of mercaptoacetic acid, methyl ester, and the procedure of Example II, but substituting the acid listed in column II for (R)-α-[[(4-ethyl-2,3-dioxo-1-piperazinyl)carbonyl]amino]benzeneacetic acid, yields the compound listed in column III. Examples 3 and 4 require a deprotection step at the end of the reaction sequence. Deprotection is accomplished in examples 3 and 4 by treatment of the protected compound with trifluoroacetic acid and anisole at −15°C.

| | Column I | Column II | Column III |
|---|---|---|---|
| 1) | 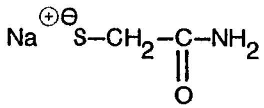 | 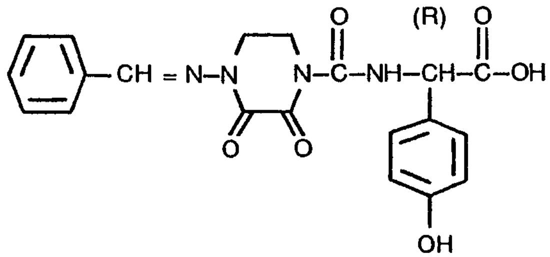 | 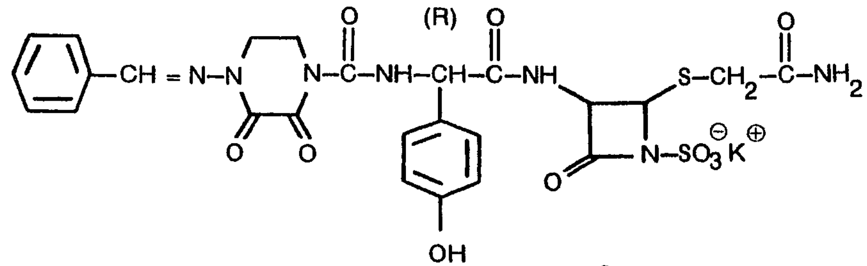 |
| 2) | 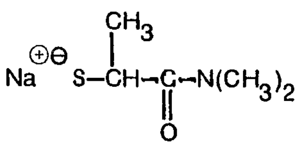 | 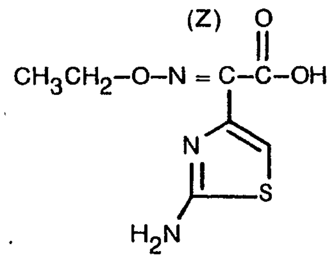 | 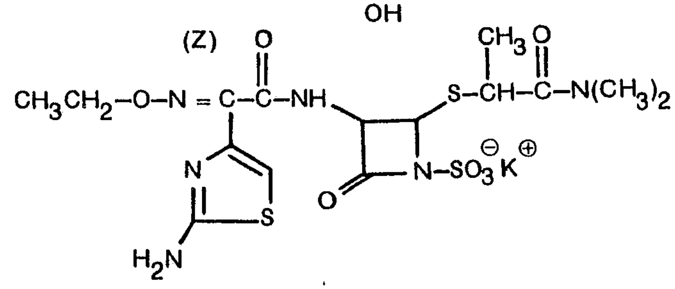 |
| 3) | 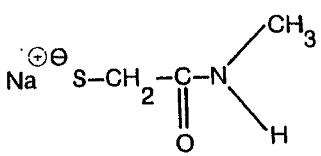 | 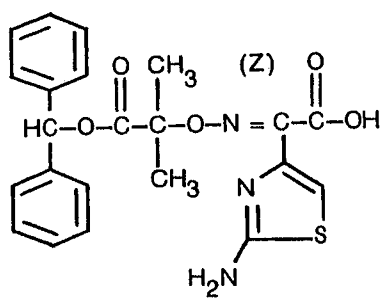 | 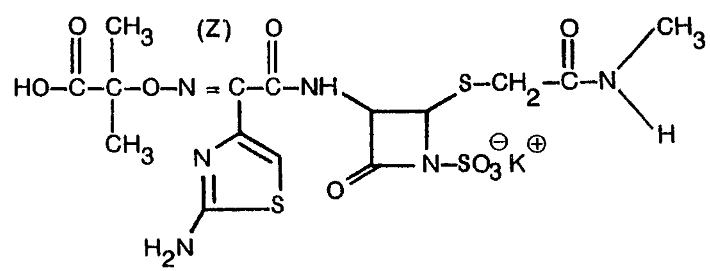 |
| 4) | 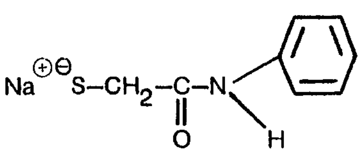 | 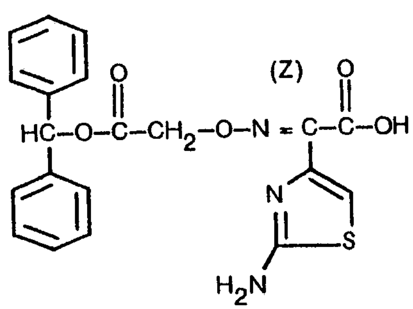 | 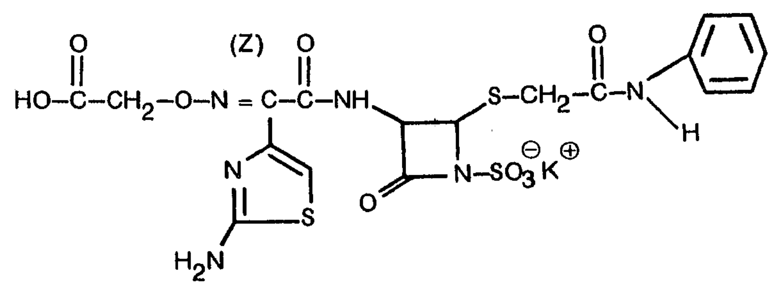 |

| | Column I | Column II | Column III |
|---|---|---|---|
| 5) | 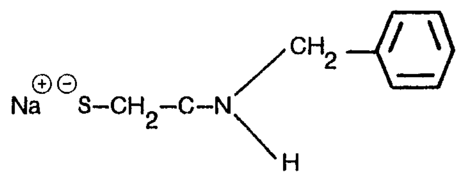 | 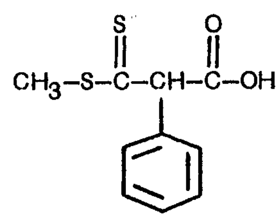 | 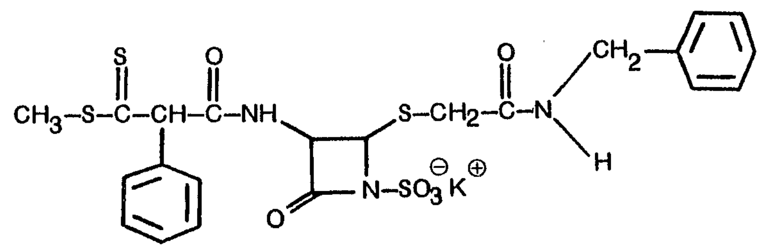 |

Examples 6—10

Following the procedure of Example III (C) and substituting therein the reactants shown in the table below, the product shown is obtained. In Examnple 6, the procedure of Example III (C) is varied by deleting treatment with potassium perfluorobutanesulfonate and instead treating first with trifluoroacetic acid to remove the diphenylmethyl protecting group and then treating with potassium-2-ethylhexaneoate to form the potassium salt.

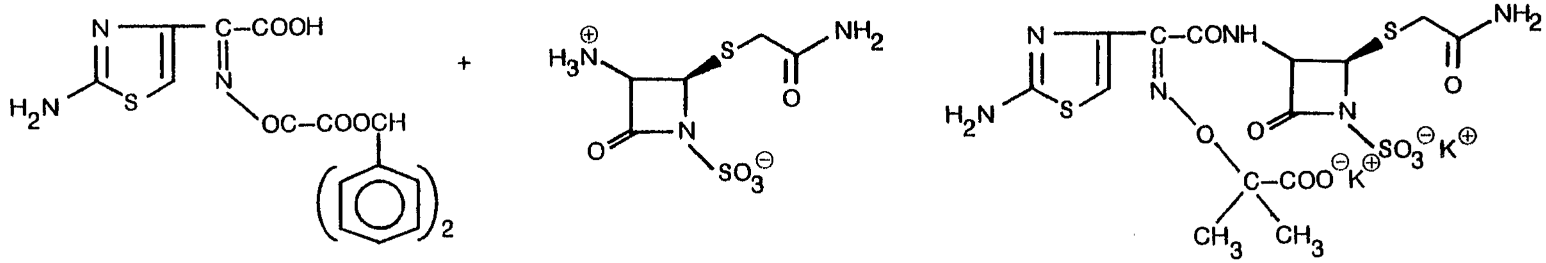

+

m.p. 215° (dec.)

(6)

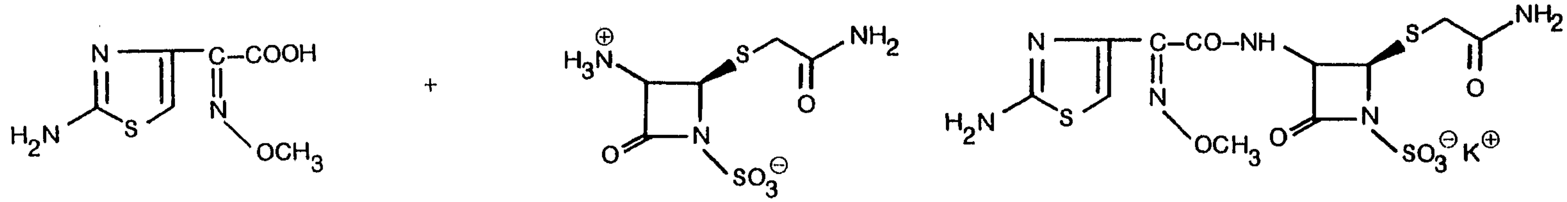

+

m.p. 186°C (dec.)

(7)

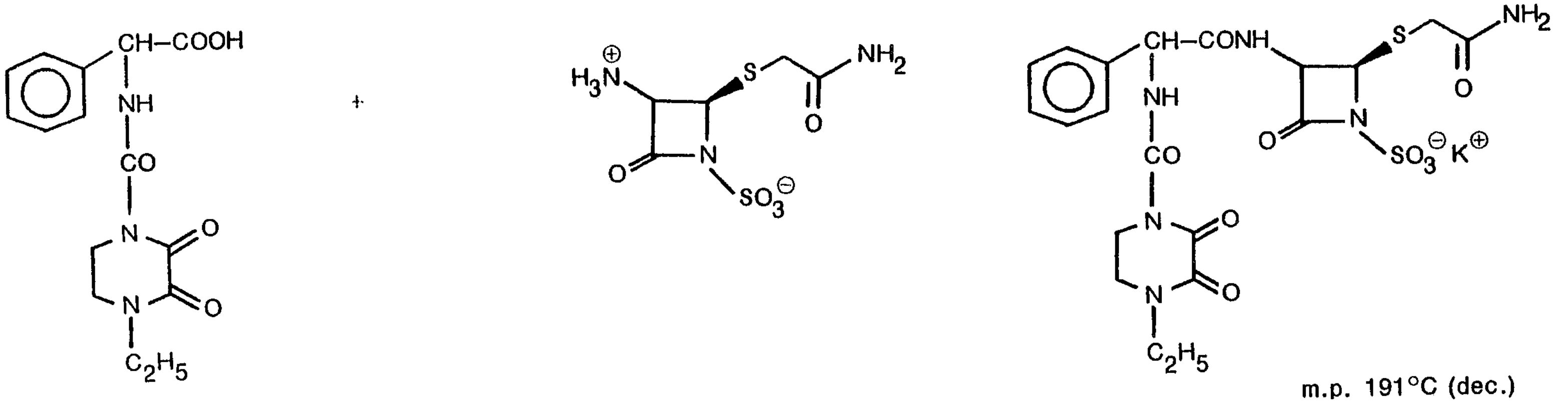

+

m.p. 191°C (dec.)

(8)

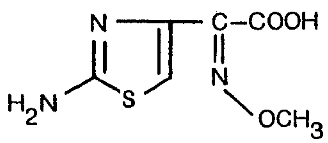
+
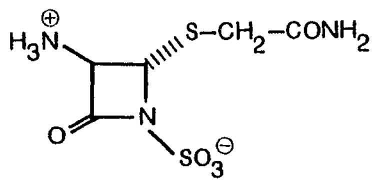
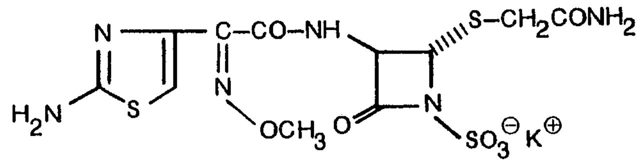
(9)

m.p. 193°C (dec.)

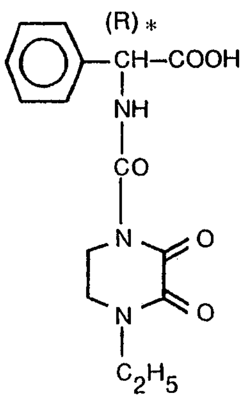
+
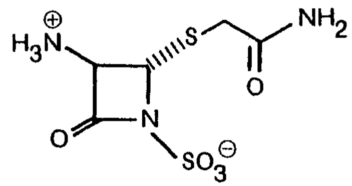
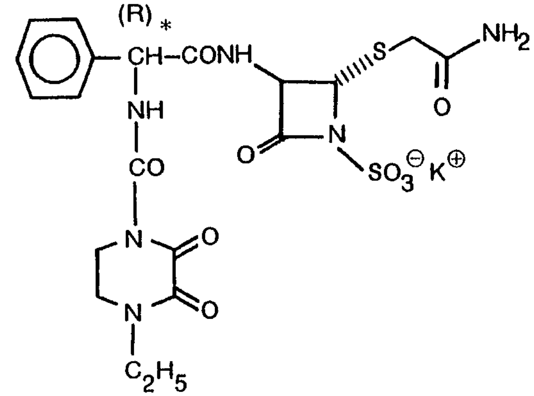
(10)

m.p. 182°C (dec.)

**Claims for the Contracting States: BE CH DE FR GB IT LI LU NL SE**

1. A compound having the formula I

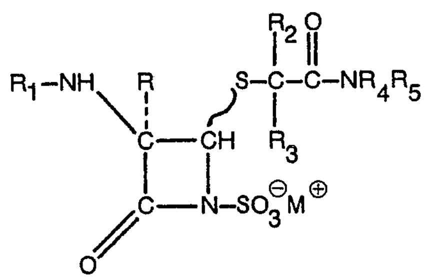

(I)

wherein R is hydrogen or methoxy;

$R_1$ is acyl, derived from a carboxylic acid, or hydrogen;

$R_2$ and $R_3$ are each independently hydrogen or $C_1$—$C_{10}$-alkyl, or $R_2$ and $R_3$ when taken together with the carbon atom to which they are attached form a $C_3$—$C_7$-cycloalkyl group;

$R_4$ and $R_5$ are the same or different and each is hydrogen, $C_1$—$C_{10}$-alkyl or phenyl or phenyl-$C_1$—$C_{10}$-alkyl wherein the phenyl group may be substituted with 1, 2 or 3 amino, halogen, hydroxyl, trifluoromethyl, $C_1$—$C_4$ alkyl or $C_1$—$C_4$-alkoxy groups;

$M^{\oplus}$ is hydrogen or a cation.

2. A compound in accordance with claim 1 wherein $R_1$ is hydrogen.

3. A compound according to claim 2 wherein $M^{\oplus}$ is hydrogen.

4. A compound in accordance with claim 1 having the structure

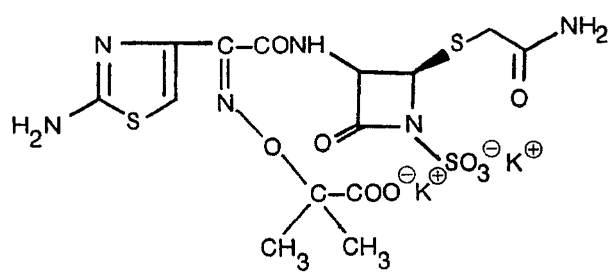

5. A compound in accordance with claim 1 having the structure

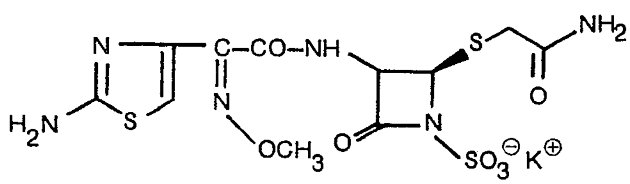

6. A compound in accordance with claim 1 having the structure

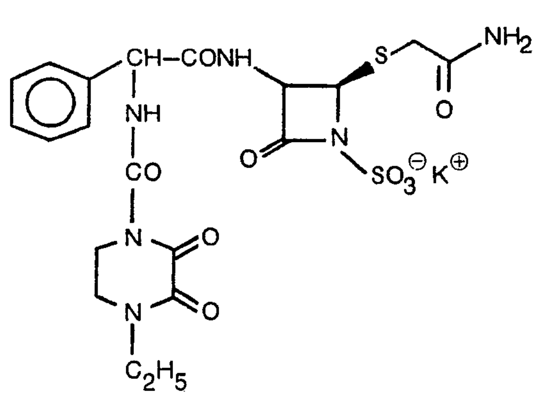

7. A compound in accordance with claim 1 having the structure

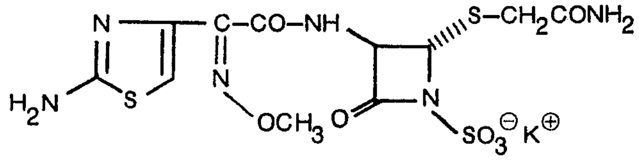

8. A compound in accordance with claim 1 having the structure

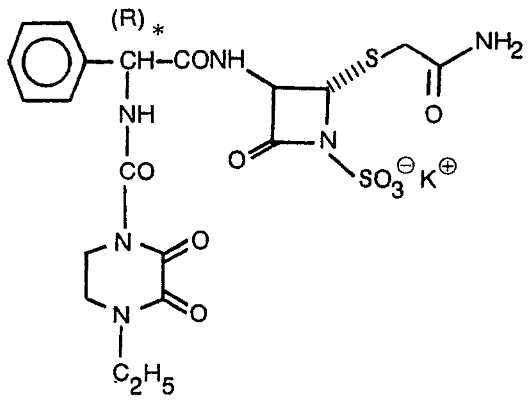

9. Use of a compound as set forth in any of claims 1 to 8 with the proviso that if $M^{\oplus}$ is hydrogen the $R_1$ group contains a basic function for the preparation of a pharmaceutical composition having an antibacterial effect.

10. A composition comprising an effective amount of a β-lactam as set forth in any of claims 1 to 8 with the proviso that if $M^{\oplus}$ is hydrogen the $R_1$ group contains a basic function; and a pharmaceutically acceptable carrier therefor.

11. A process for preparing a compound as set forth in any of claims 1 to 8, characterized by sulfonating a compound of the formula

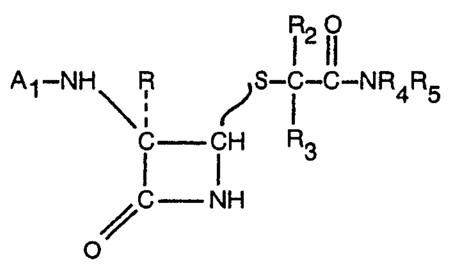

wherein $A_1NH$— is amino or a protected amino group and R, $R_2$, $R_3$, $R_4$ and $R_5$ are as defined in claim 1, to yield a compound of the formula

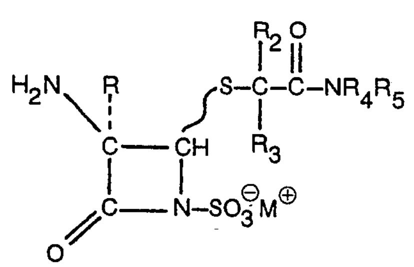

wherein $M^{\oplus}$ is as defined in claim 1, which compound may be acylated to introduce the $R_1$-group, being an acyl as defined in claim 1.

**Claims for the Contracting State: AT**

1. A process for preparing a compound having the formula I

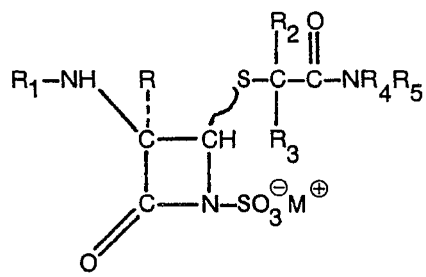

(I)

wherein R is hydrogen or methoxy;

$R_1$ is acyl, derived from a carboxylic acid, or hydrogen;

$R_2$ and $R_3$ are each independently hydrogen or $C_1$—$C_{10}$-alkyl, or $R_2$ and $R_3$ when taken together with the carbon atom to which they are attached form a $C_3$—$C_7$-cycloalkyl group;

$R_4$ and $R_5$ are the same or different and each is hydrogen, $C_1$—$C_{10}$-alkyl or phenyl or phenyl-$C_1$—$C_{10}$-alkyl wherein the phenyl group may be substituted with 1, 2 or 3 amino, halogen, hydroxyl, trifluoromethyl, $C_1$—$C_4$ alkyl or $C_1$—$C_4$-alkoxy groups;

$M^{\oplus}$ is hydrogen or a cation, characterized by sulfonating a compound of the formula

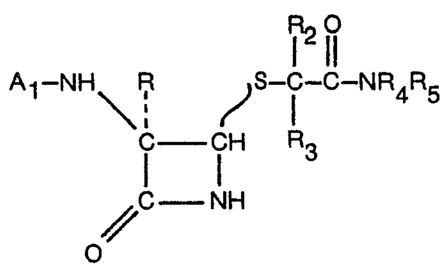

wherein $A_1NH$— is amino or a protected amino group and R, $R_2$, $R_3$, $R_4$ and $R_5$ are as defined above, to yield a compound of the formula

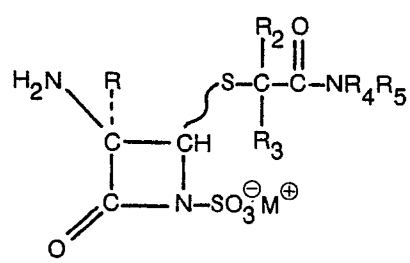

wherein $M^{\oplus}$ is as defined above, which compound may be acylated to introduce the $R_1$-group, being an acyl as defined above.

2. A process for preparing a compound in accordance with claim 1 wherein $R_1$ is hydrogen.
3. A process for preparing a compound according to claim 2 wherein $M^{\oplus}$ is hydrogen.
4. A process for preparing the compound of claim 1 having the structure

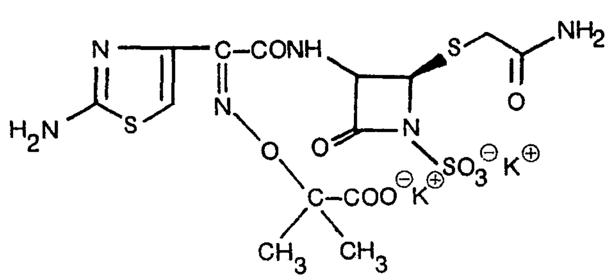

5. A process for preparing the compound of claim 1 having the structure

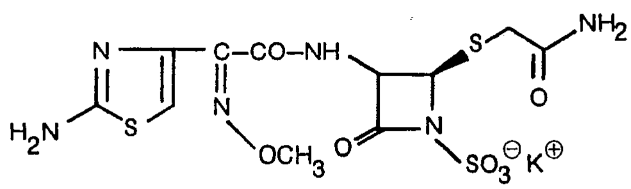

6. A process for preparing the compound of claim 1 having the structure

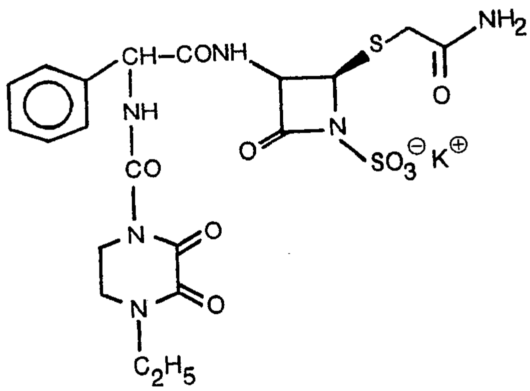

7. A process for preparing the compound of claim 1 having the structure

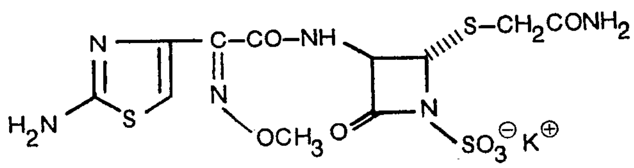

8. A process for preparing the compound of claim 1 having the structure

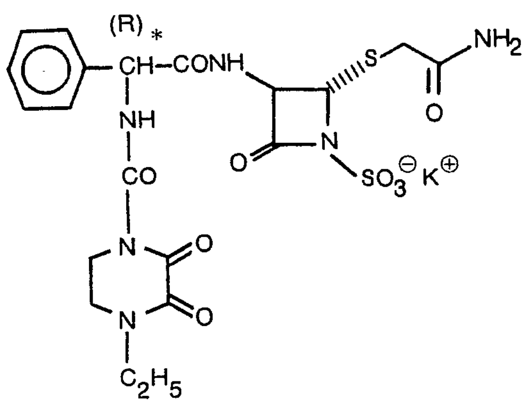

9. A process for preparing a composition comprising the formulation of an effective amount of a β-lactam as set forth in claim 1, with the proviso that if $M^{\oplus}$ is hydrogen the $R_1$ group contains a basic function, with a pharmaceutically acceptable carrier therefor.

**Patentansprüche für die Vertragsstaaten: BE CH DE FR GB IT LI LU NL SE**

1. Verbindung der allgemeinen Formel I

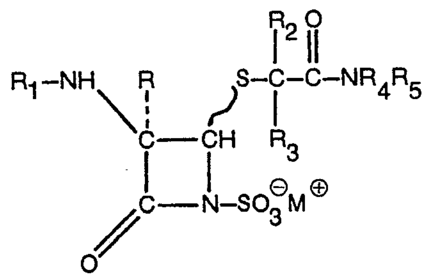

(I)

in der R ein Wasserstoffatom oder eine Methoxygruppe bedeutet und

$R_1$ ein von einer Carbonsäure abgeleiteter Acylrest oder ein Wasserstoffatom ist;

$R_2$ und $R_3$ unabhängig voneinander jeweils ein Wasserstoffatom oder ein $C_1$—$C_{10}$-Alkylrest sind, oder $R_2$ und $R_3$ zusammen mit dem Kohlenstoffatom, an das sie gebunden sind, einen $C_3$—$C_7$-Cycloalkylrest bilden;

$R_4$ und $R_5$ gleich oder unterschiedlich sind und jeweils ein Wasserstoffatom, einen $C_1$—$C_{10}$-Alkylrest oder eine Phenylgruppe oder einen Phenyl-$C_1$—$C_{10}$-alkylrest bedeuten, wobei die Phenylgruppe gegebenenfalls mit 1, 2 oder 3 Amino-, Halogen-, Hydroxyl-, Trifluormethyl-Gruppen, $C_1$—$C_4$-Alkyl- oder $C_1$—$C_4$-Alkoxyresten substituiert ist; und

$M^{\oplus}$ ein Wasserstoffatom oder ein Kation ist.

2. Verbindung nach Anspruch 1, in der $R_1$ ein Wasserstoffatom ist.

3. Verbindung nach Anspruch 2, in der $M^{\oplus}$ ein Wasserstoffatom ist.

4. Verbindung nach Anspruch 1 mit der Formel

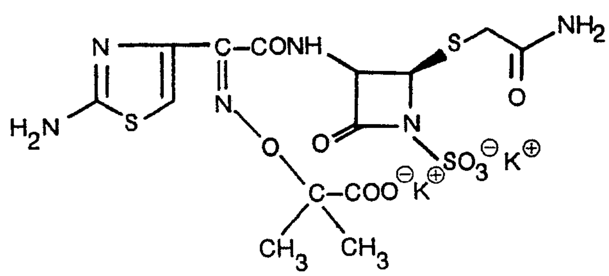

5. Verbindung nach Anspruch 1 mit der Formel

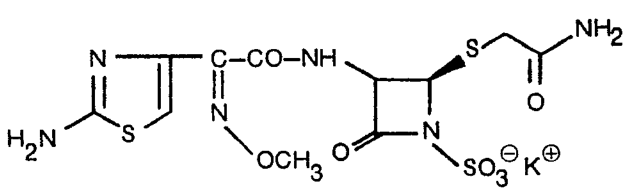

6. Verbindung nach Anspruch 1 mit der Formel

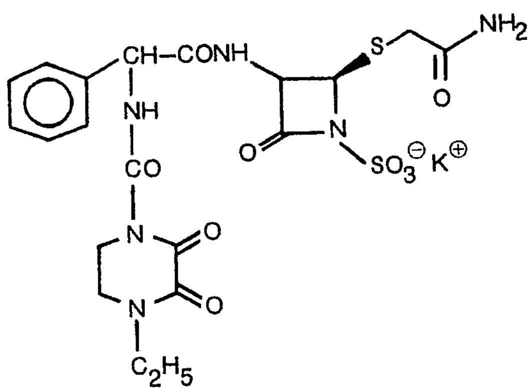

7. Verbindung nach Anspruch 1 mit der Formel

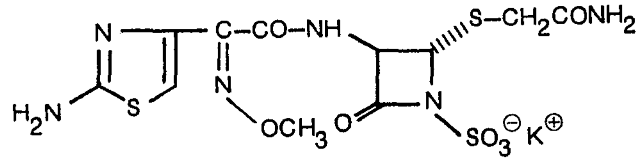

8. Verbindung nach Anspruch 1 mit der Formel

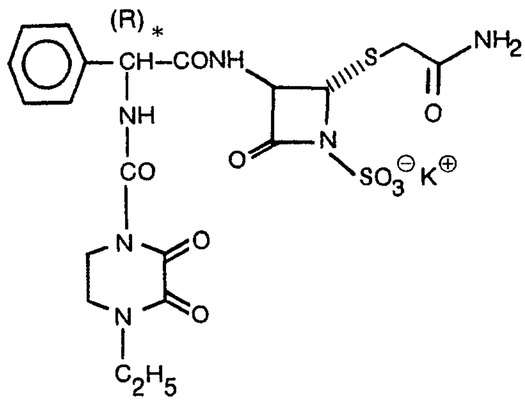

9. Verwendung einer Verbindung nach einem der Ansprüche 1 bis 8 zur Herstellung eines Arzneimittels mit einer antibakteriellen Wirkung, wobei der Rest $R_1$ eine basische Funktion enthält, falls $M^{\oplus}$ ein Wasserstoffatom ist.

10. Mittel, enthaltend eine wirksame Menge eines β-Lactams nach einem der Ansprüche 1 bis 8 und einen pharmazeutisch verträglichen Träger dafür, wobei der Rest $R_1$ eine basische Funktion enthält, falls $M^{\oplus}$ ein Wasserstoffatom ist.

11. Verfahren zur Herstellung einer Verbindung nach einem der Ansprüche 1 bis 8, dadurch gekennzeichnet, daß man eine Verbindung der allgemeinen Formel

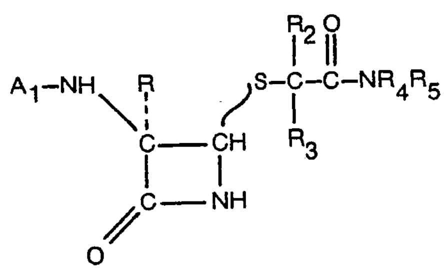

sulfoniert, in der $A_1NH$— eine Amino- oder eine geschützte Aminogruppe ist und R, $R_2$, $R_3$, $R_4$ und $R_5$ die im Anspruch 1 angegebene Bedeutung haben, und dabei eine Verbindung der allgemeinen Formel

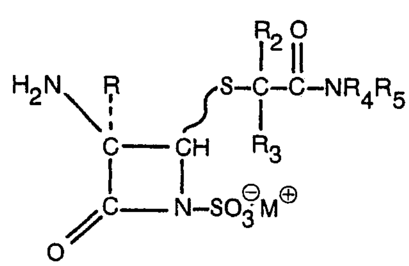

erhält, in der $M^{\oplus}$ die in Anspruch 1 angegebene Bedeutung hat, die man gegebenenfalls acyliert, um die Gruppe $R_1$, die eine Acylgruppe mit der in Anspruch 1 angegebenen Bedeutung ist, einzuführen.

**Patentansprüche für den Vertragsstaat: AT**

1. Verfahren zur Herstellung einer Verbindung der allgemeinen Formel I

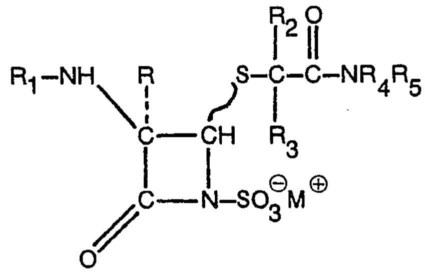

(I)

in der R ein Wasserstoffatom oder eine Methoxygruppe bedeutet und

$R_1$ ein von einer Carbonsäure abgeleiteter Acylrest oder ein Wasserstoffatom ist;

$R_2$ und $R_3$ unabhängig voneinander jeweils ein Wasserstoffatom oder ein $C_1$—$C_{10}$-Alkylrest sind, oder $R_2$ und $R_3$ zusammen mit dem Kohlenstoffatom, an das sie gebunden sind, einen $C_3$—$C_7$-Cycloalkylrest bilden;

$R_4$ und $R_5$ gleich oder unterschiedlich sind und jeweils ein Wasserstoffatom, einen $C_1$—$C_{10}$-Alkylrest oder eine Phenylgruppe oder einen Phenyl-$C_1$—$C_{10}$-alkylrest bedeuten, wobei die Phenylgruppe gegebenenfalls mit 1, 2 oder 3 Amino-, Halogen-, Hydroxyl-, Trifluormethyl-Gruppen, $C_1$—$C_4$-Alkyl- oder $C_1$—$C_4$-Alkoxyresten substituiert ist; und

$M^{\oplus}$ ein Wasserstoffatom oder ein Kation ist, dadurch gekennzeichnet, daß man eine Verbindung der allgemeinen Formel

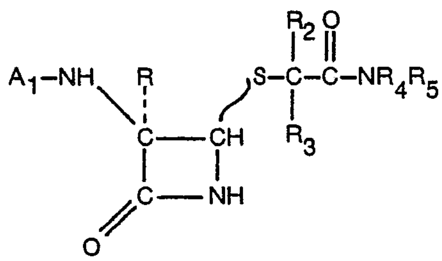

sulfoniert, in der $A_1NH$— eine Amino- oder eine geschützte Aminogruppe ist und R, $R_2$, $R_3$, $R_4$ und $R_5$ die oben angegebene Bedeutung haben, und dabei eine Verbindung der allgemeinen Formel

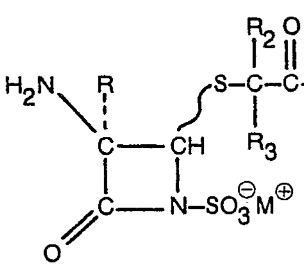

erhält, in der $M^{\oplus}$ die oben angegebene Bedeutung hat, die man gegebenenfalls acyliert, um die Gruppe $R_1$, die eine Acylgruppe mit der oben angegebenen Bedeutung ist, einzuführen.

2. Verfahren zur Herstellung einer Verbindung nach Anspruch 1, in der $R_1$ ein Wasserstoffatom ist.

3. Verfahren zur Herstellung einer Verbindung nach Anspruch 2, in der $M^{\oplus}$ ein Wasserstoffatom ist.

4. Verfahren zur Herstellung der Verbindung nach Anspruch 1 mit der Formel

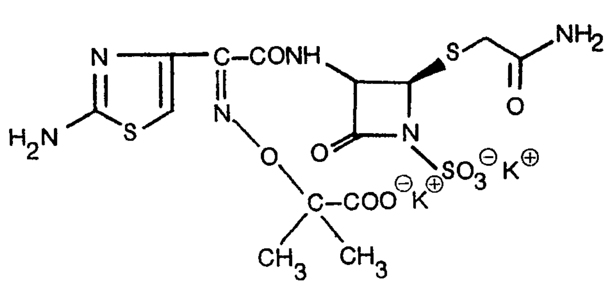

5. Verfahren zur Herstellung der Verbindung nach Anspruch 1 mit der Formel

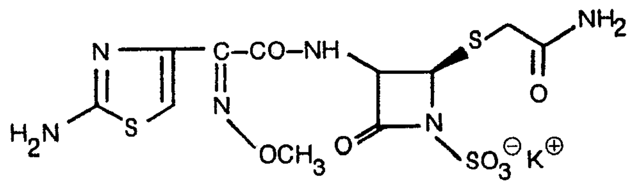

6. Verfahren zur Herstellung der Verbindung nach Anspruch 1 mit der Formel

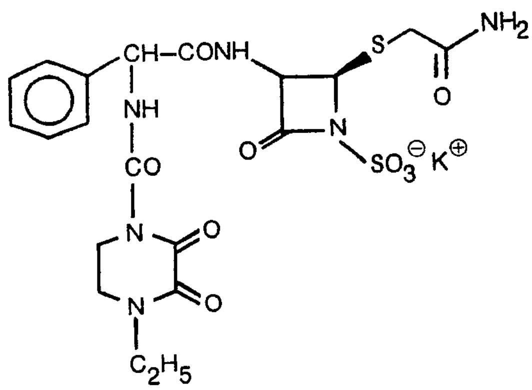

7. Verfahren zur Herstellung der Verbindung nach Anspruch 1 mit der Formel

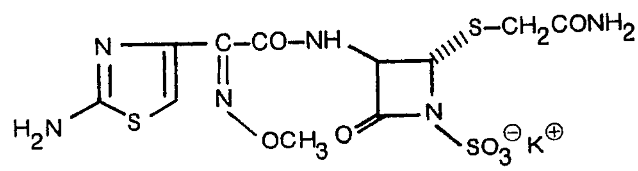

8. Verfahren zur Herstellung der Verbindung nach Anspruch 1 mit der Formel

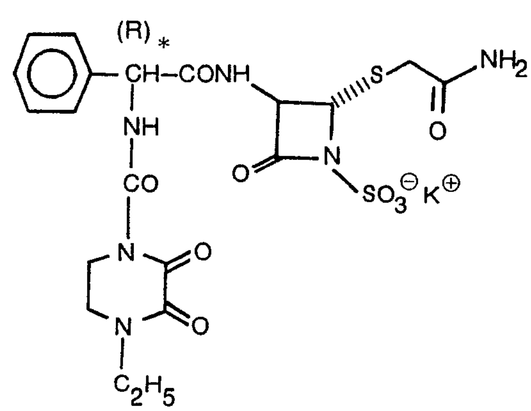

9. Verfahren zur Herstellung eines Mittels enthaltend die Formulierung einer wirksamen Menge eines β-Lactams nach Anspruch 1 mit einem pharmazeutisch verträglichen Träger dafür, wobei der Rest $R_1$ eine basische Funktion enthält, falls $M^{\oplus}$ ein Wasserstoffatom ist.

**Revendications pour les Etats contractants: BE CH DE FR GB IT LI LU NL SE**

1. Composé de formule I

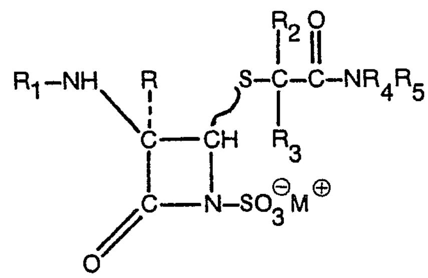

(I)

dans laquelle R est un atome d'hydrogène ou un radical méthoxy;

$R_1$ est un radical acyle dérivé d'un acide carboxylique, ou un atome d'hydrogène;

$R_2$ et $R_3$ sont chacun, indépendamment, un atome d'hydrogène ou un radical alkyle en $C_1$—$C_{10}$ ou bien $R_2$ et $R_3$ forment, avec l'atome de carbone auquel ils sont fixés, un radical cycloalkyle en $C_3$—$C_7$;

$R_4$ et $R_5$ sont identiques ou différents et sont chacun un atome d'hydrogène, un radical alkyle en $C_1$—$C_{10}$ ou phényle ou phényl-alkyle en $C_1$—$C_{10}$ dans lequel le radical phényle peut être substitué par 1, 2 ou 3 atomes ou groupements amine, halogène, hydroxyle, trifluorométhyle, alkyle en $C_1$—$C_4$ ou alcoxy en $C_1$—$C_4$;

$M^{\oplus}$ est un atome d'hydrogène ou un cation.

2. Composé selon la revendication 1, dans lequel $R_1$ est un atome d'hydrogène.

3. Composé selon la revendication 2, dans lequel $M^{\oplus}$ est un atome d'hydrogène.

4. Composé selon la revendication 1, ayant pour formule développée

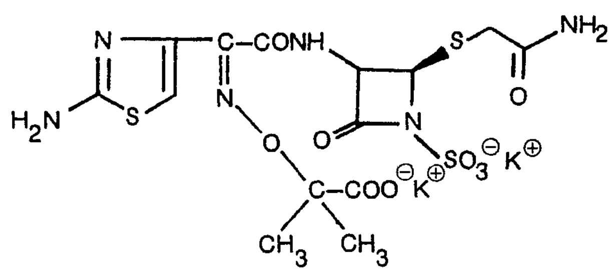

5. Composé selon la revendication 1, ayant pour formule développée

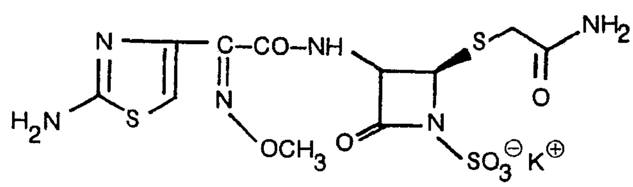

6. Composé selon la revendication 1, ayant pour formule développée

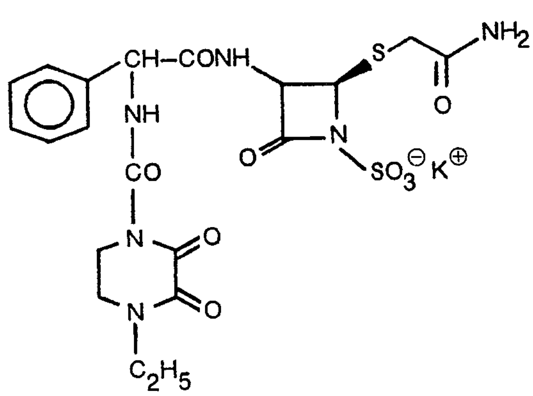

7. Composé selon la revendication 1, ayant pour formule développée

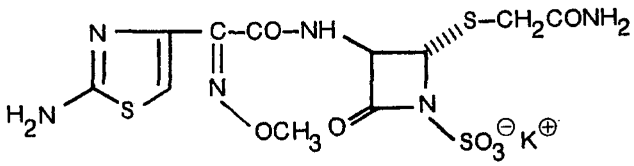

8. Composé selon la revendication 1, ayant pour formule développée

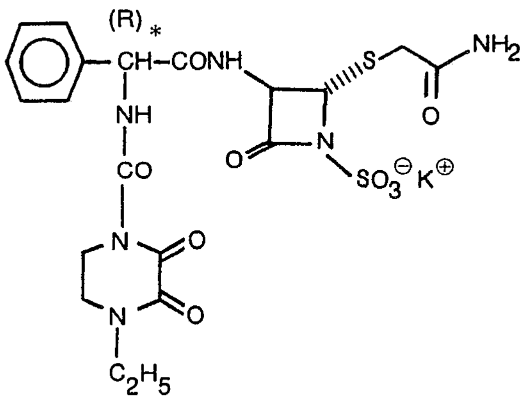

9. Utilisation d'un composé tel qu'il est indiqué dans l'une quelconque des revendications 1 à 8, à cette condition que, si $M^{\oplus}$ est un atome d'hydrogène, le groupement $R_1$ contient une fonction basique, pour la préparation d'une composition pharmaceutique ayant un effet antibactérien.

10. Composition comprenant une quantité efficace d'un β-lactame tel qu'il est indique dans l'une quelconque des revendications 1 à 8, à cette condition que, si $M^{\oplus}$ est un atome d'hydrogène, le groupement $R_1$ contient une fonction basique;

et un porteur pharmaceutiquement acceptable pour celui-ci.

11. Procédé de préparation d'un composé tel qu'il est indiqué dans l'une quelconque des revendications 1 à 8, caractérisé en ce qu'on sulfone un composé de formule

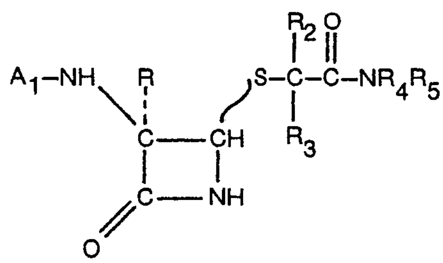

dans laquelle $A_1NH$— est un groupement amine ou un groupement amine protégé et R, $R_2$, $R_3$, $R_4$ et $R_5$ sont tel que définis dans la revendication 1, pour obtenir un composé de formule

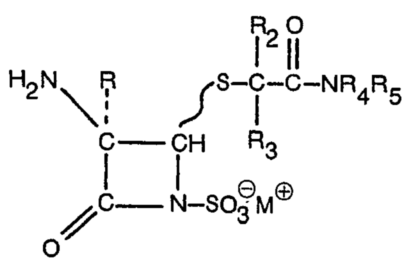

dans laquelle $M^{\oplus}$ est tel que défini dans la revendication 1, composé que l'on peut acyler pour introduire le groupement $R_1$, celui-ci étant un radical acyle tel qu'il est défini dans la revendication 1.

**Revendications pour l'Etat contractant: AT**

1. Procédé de préparation d'un composé de formule

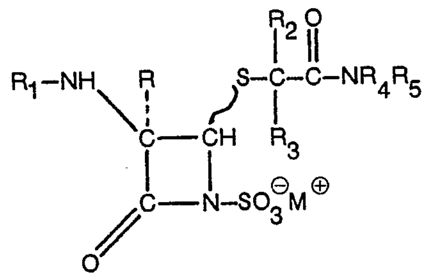

(I)

dans laquelle R est un atome d'hydrogène ou un radical méthoxy;

$R_1$ est un radical acyle dérivé d'un acide carboxylique, ou un atome d'hydrogène;

$R_2$ et $R_3$ sont chacun, indépendamment, un atome d'hydrogène ou un radical alkyle en $C_1$—$C_{10}$ ou bien $R_2$ et $R_3$ forment, avec l'atome de carbone auquel ils sont fixés, un radical cycloalkyle en $C_3$—$C_7$;

$R_4$ et $R_5$ sont identiques ou différents et sont chacun un atome d'hydrogène, un radical alkyle en $C_1$—$C_{10}$ ou phényle ou phényl-alkyle en $C_1$—$C_{10}$ dans lequel le radical phényle peut être substitué par 1, 2 ou 3 atomes ou groupements amine, halogène, hydroxyle, trifluorométhyle, alkyle en $C_1$—$C_4$ ou alcoxy en $C_1$—$C_4$;

$M^{\oplus}$ est un atome d'hydrogène ou un cation, caractérisé en ce qu'on sulfone une composé de formule

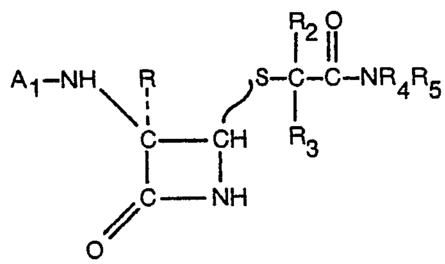

dans laquelle $A_1NH$ est un groupement amine ou un groupement amine protégé et R, $R_2$, $R_3$, $R_4$ et $R_5$ sont tel que définis ci-dessus, pour obtenir un composé de formule

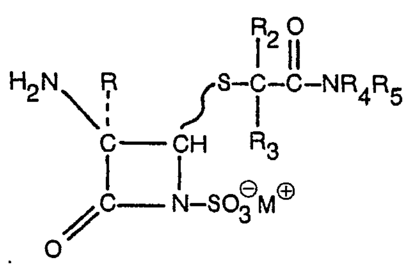

dans laquelle $M^{\oplus}$ est tel que défini ci-dessus, composé que l'on peut acyler pour introduire le groupement $R_1$, celui-ci étant un radical acyle tel qu'il est défini ci-dessus.

2. Procédé de préparation d'un composé selon la revendication 1, dans lequel $R_1$ est un atome d'hydrogène.

3. Procédé de préparation d'un composé selon la revendication 2, dans lequel $M^{\oplus}$ est un atome d'hydrogène.

4. Procédé de préparation du composé de la revendication 1, ayant pour formule développée

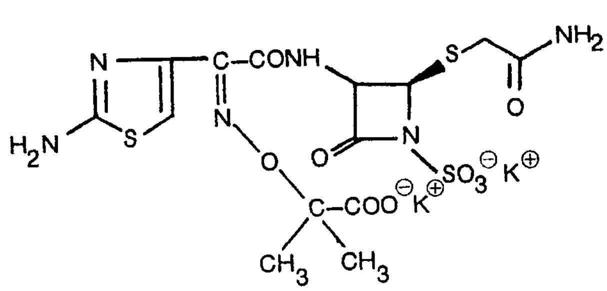

5. Procédé de préparation du composé de la revendication 1, ayant pour formule développée

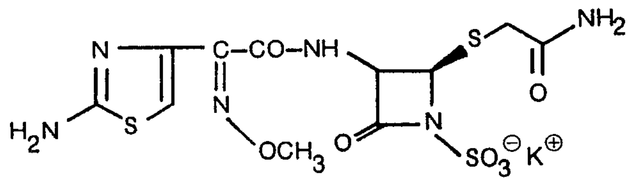

6. Procédé de préparation du composé de la revendication 1, ayant pour formule développée

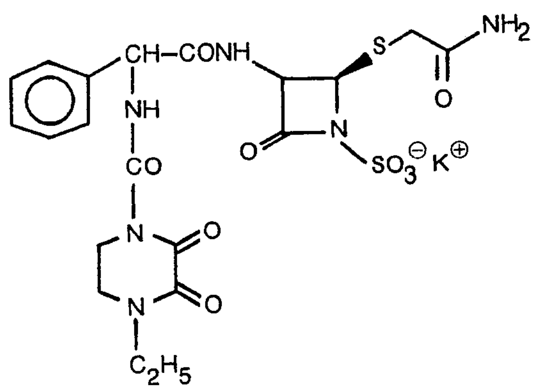

7. Procédé de préparation du composé de la revendication 1, ayant pour formule développée

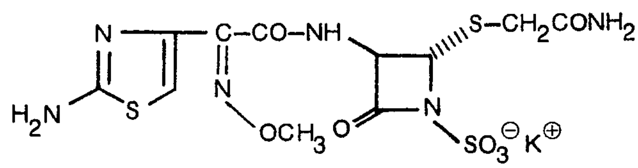

8. Procédé de préparation du composé de la revendication 1, ayant pour formule développée

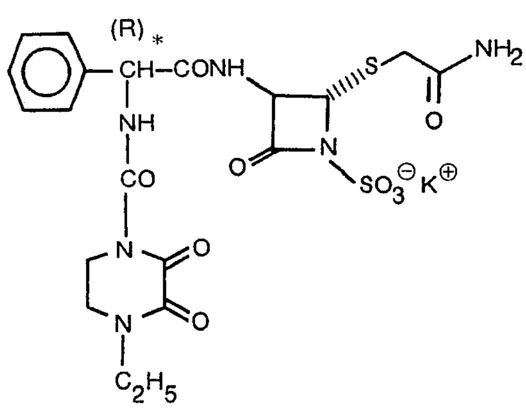

9. Procédé de préparation d'une composition comprenant la formulation d'une quantité efficace d'un β-lactame tel qu'il est indiqué dans la revendication 1, à cette condition qui, si $M^{\oplus}$ est un atome d'hydrogène, le groupement $R_1$ contient une fonction basique, avec un porteur pharmaceutiquement acceptable pour ce β-lactame.